# EUROPÄISCHE PATENTSCHRIFT

(11) **EP 2 498 855 B1**
(45) Veröffentlichungstag und Bekanntmachung des Hinweises auf die Patenterteilung: **22.03.2017**
(21) Anmeldenummer: 10751595.9
(22) Anmeldetag: 27.08.2010
(51) Int. Cl.: A61M 16/12, A61M 16/00

(54) **DRUCKLUFTSTEUERUNGSVORRICHTUNG FÜR EINE CPAP-VORRICHTUNG UND ENTSPRECHENDES CPAP-SYSTEM**
COMPRESSED AIR CONTROL DEVICE FOR A CPAP DEVICE AND CORRESPONDING CPAP SYSTEM
DISPOSITIF DE COMMANDE À AIR COMPRIMÉ POUR UN DISPOSITIF CPAP ET SYSTÈME CPAP CORRESPONDANT

(30) Priorität: 09.11.2009 DE 102009046541
(43) Veröffentlichungstag der Anmeldung: 19.09.2012
(73) Patentinhaber: MEDIN MEDICAL INNOVATIONS GMBH, 82140 Olching (DE)
(72) Erfinder: KNIEWASSER, Gert, 86926 Greifenberg (DE)
(74) Vertreter: Isarpatent
(86) Internationale Anmeldenummer: PCT/EP2010/062572
(87) Internationale Veröffentlichungsnummer: WO 2011/054556

(56) Entgegenhaltungen:
- EP-A1- 1 161 963
- US-A- 4 401 115
- US-A1- 2004 020 488
- US-A1- 2009 126 731

## Beschreibung

### Stand der Technik

Die vorliegende Erfindung betrifft eine Druckluftsteuerungsvorrichtung für eine CPAP-Vorrichtung, wie aus der US 2009/0126731 A1 oder der EP 1 161 963 A1 bekannt.

Aus der US 4,401,115 ist es bekannt, Druckimpulse zur Unterstützung der Atmung eines Patienten mit natürlichen Atemreflexen zu korrelieren.

Das Prinzip einer Vorrichtung zur Erzeugung eines kontinuierlichen positiven Atemwegdrucks oder kurz CPAP-Vorrichtung, und insbesondere einer nasalen CPAP-Vorrichtung oder kurz nCPAP-Vorrichtung, besteht darin, dass ein damit behandelter Patient mit Druckunterstützung ein- und ausatmet.

Eine äußerst wichtige Anwendung liegt in der Frühgeborenenbehandlung. Der Effekt dabei ist, dass die noch nicht ausgereifte Lunge durch den Überdruck aufgebläht wird und dadurch ein besserer Gasaustausch möglich ist. Ein weiterer sehr bedeutender Faktor ist, dass das gefährliche Kollabieren der Alveolen durch den Atmungsunterdruck, durch diese Technik verhindert werden kann. Noch ein Gesichtspunkt ist, dass die Alveolen besser geöffnet werden, und somit der gesamte Funktionszustand der Lunge verbessert wird.

Fig.,3 ist eine schematische Darstellung der aus der WO 99/24101 bekannten Vorrichtung 1 zur Erzeugung eines kontinuierlichen positiven Atemwegdrucks.

Bezugszeichen 10 bezeichnet einen zylindrischer Hohlkörper 10, in dem ein Überdruck aufbaubar ist und Bezugszeichen 20 eine in der Mantelfläche des zylindrischen Hohlkörpers 10 vorgesehene erste Öffnung zum Zuführen eines in den Hohlkörper 10 gerichteten konstanten Druckluftstroms A und zum Abführen des ausgeatmeten Luftstroms B. Weiterhin vorgesehen sind ein am zylindrischen Hohlkörper 10 an dessen einer Stirnfläche 10a anbringbares Anschlußstück 30 zur Verbindung des Hohlkörpers 10 mit einem Nasenansatzstück 100 und ein am Hohlkörper 10 anbringbarer Abstandshalter 40, an dem eine Strömungsdüse 50 zum Richten des konstanten Druckluftstroms A auf die erste Öffnung 20 anbringbar ist. Die Strömungsdüse weist eine Anschlußröhre L4b zum Anbringen einer Druckluftleitung zum Zuführen des konstanten Druckluftstroms A auf.

Das Anschlußstück 30 besteht aus einem Stopfen aus Teflon, der an der Stirnfläche 10a in den Hohlkörper 10 zumindest teilweise einführbar ist. Er weist zwei Durchführungen auf (gestrichelte Linien), welche entsprechenden Atemluftwegen des Nasenansatzstücks 100 entsprechen. In den Durchführungen des Anschlußstücks 30 sind zwei nach außen weisende Röhrenstummel 35 zum Einführen in das Nasenansatzstück 100 vorgesehen.

An der anderen Stirnfläche 10b des zylindrischen Hohlkörpers 10 ist eine zweite Öffnung 02 zum Anschließen eines Druckmeßgeräts vorgesehen. Dazu ist mit der zweiten Öffnung 02 unter einem im wesentlichen rechtem Winkel eine Röhre 60 zum Anschluß an eine Druckmeßleitung verbunden. Die Röhre 60 erstreckt sich im wesentlichen um die gleiche Länge vom Hohlkörper 10 weg wie die Strömungsdüse 50, so dass eine Leitung zur Zuführung von Druckluft an die Strömungsdüse im wesentlichen auf gleicher Höhe wie die Druckmeßleitung an die Strömungsdüse 50 anbringbar ist.

Der Abstandshalter 40 weist eine im wesentlichen ringförmige Gestalt auf. Die Strömungsdüse 50 ist durch ein Loch in der Seitenwand der Ringgestalt geführt und im wesentlichen senkrecht auf die erste Öffnung 20 gerichtet. Die Strömungsdüse 50 ragt um eine vorbestimmte Länge in das Ringgestaltinnere hinein.

Die Seitenwand der Ringgestalt weist eine Sicherheits-Luftdurchtrittsöffnung 45 auf. Die Ringgestalt dieser Konstruktion ist mit den Fingern derart verschließbar, dass der ausgeatmete Luftstrom B durch die Sicherheits-Luftdurchtrittsöffnung 45 verläuft.

Die dieser bekannten Vorrichtung zur Erzeugung eines kontinuierlichen positiven Atemwegdrucks zugrundeliegende Idee besteht darin, dass ein Benveniste-Ventil vorgesehen ist, dessen Hohlraum durch Abnehmen des Anschlußstücks von einer Seite her über einen großen Querschnitt zugänglich gemacht werden kann, so dass eine effektive Sterilisation des Hohlraums möglich ist. Im Betrieb ist kein Ausatmungsschlauch notwendig.

Ein Nachteil der bekannten CPAP- bzw. nCPAP-Vorrichtungen in der Tatsache, dass sie nicht flexibel in der Handhabung der Druckluft und im Betrieb mit Druckluft-Erzeugungs-einrichtungen und Druckmeßgeräten sind.

### VORTEILE DER ERFINDUNG

Die erfindungsgemäße Druckluftsteuerungsvorrichtung für eine CPAP-Vorrichtung mit den Merkmalen des Anspruchs 1 und das entsprechende CPAP-System nach Anspruch 8 weisen den Vorteil auf, dass bei CPAP-Behandlungen flexibler auf den Zustand des Patienten reagiert werden kann, welcher sich im analysierten Drucksignal der CPAP-Vorrichtung widerspiegelt.

So ist es beispielsweise möglich, Druckimpulse auszugeben, welche mit natürlichen Atemreflexen korreliert bzw. synchronisiert sind.

Zudem lassen sich beispielsweise lebensbedrohliche Apnoephasen ausgesetzter Atemtätigkeit erkennen, auf die mit einem Druckimpuls zur Stimulation der natürlichen Atmung reagiert werden kann.

Besonders vorteilhaft ist dabei, dass über den Hohlkörper der CPAP-Vorrichtung, welche nach dem Benviste-Prinzip funktioniert, die Atemtätigkeit direkt erfasst werden kann.

In den Unteransprüchen finden sich vorteilhafte Weiterbildungen und Verbesserungen des jeweiligen Gegenstandes der Erfindung.

Gemäß einer bevorzugten Weiterbildung ist die Druckverlauf-Auswerteeinrichtung zum Erzeugen eines Fluß-Steuersignals basierend auf dem Resultat des Auswertens gestaltet, wobei eine Fluß-Steuervorrichtung vorgesehen ist, welche derart gestaltet ist, dass sie ansprechend auf das Fluß-Steuersignal einen Fluß des im wesentlichen konstanten Druckluftstroms steuert. Beispielsweise könnte analog wie beim Druck ein Grundfluß vorgesehen werden, der entsprechend dem Fluß-Steuersignal moduliert wird.

Gemäß einer weiteren bevorzugten Weiterbildung umfasst die Druckverlauf-Auswerteeinrichtung eine Speichereinrichtung, in der Referenz-Druckverläufe gespeichert sind, und die Druckverlauf-Auswerteeinrichtung derart gestaltet ist, dass sie beim Auswerten ein Vergleichen mit den Referenz-Druckverläufen durchführt.

Gemäß einer weiteren bevorzugten Weiterbildung ist die Fluß-Steuervorrichtung von einer Luft-Versorgungseinrichtung und von einer Sauerstoffversorgungseinrichtung gespeist und derart gestaltet, dass durch sie ein Konzentrationsverhältnis von Luft und Sauerstoff einstallbar ist.

Erfindungsgemäß ist die Druckverlauf-Auswerteeinrichtung derart gestaltet, dass sie beim Auswerten eine Apnoephase erfasst und ansprechend auf eine derartige Erfassung ein Druckmodulations-Steuersignal zum Auslösen eines Druckimpulses im modulierten Druckluftstrom an die Druckmodulationsvorrichtung ausgibt.

Erfindungsgemäß erfasst die Druckverlauf-Auswerteeinrichtung zum Erkennen einer Apnoephase, ob der erfasste Druck im Hohlkörper eine vorbestimmte Zeitdauer im wesentlichen konstant ist, wobei die vorbestimmte Zeitdauer vorzugsweise im Bereich von 2 bis 5s liegt.

Erfindungsgemäß ist die Druckverlauf-Auswerteeinrichtung derart gestaltet, dass sie beim Auswerten natürliche Atemreflexe erfasst und ansprechend auf eine derartige Erfassung ein Druckmodulations-Steuersignal zum Auslösen eines Druckimpulses im modulierten Druckluftstrom an die Druckmodulationsvorrichtung ausgibt, welcher mit dem natürlichen Atemreflex korreliert und synchronisiert ist.

Gemäß einer weiteren bevorzugten Weiterbildung erfasst die Druckverlauf-Auswerteeinrichtung zum Erkennen des Einsetzens natürlicher Atemreflexe, ob der Druck eine vorbestimmte abfallende Flanke aufweist.

Gemäß einer weiteren bevorzugten Weiterbildung ist die Druckmodulationsvorrichtung derart gestaltet, dass durch sie eine vorgebbare Grundmodulation im modulierten Druck-luftstrom zusätzlich vorsehbar ist, insbesondere eine periodische Modulation.

Gemäß einer weiteren bevorzugten Weiterbildung ist der jeweilge Druckimpuls im wesentlichen rechteckförmig.

### ZEICHNUNGEN

Ausführungsbeispiele der Erfindung sind in den Zeichnungen dargestellt und in der nachfolgenden Beschreibung näher erläutert.

Es zeigen:
- Fig. 1: eine schematische Darstellung einer Ausführungsform des erfindungsgemäßen CPAP-Systems mit einer entsprechenden Ausführungsform der erfindungsgemäßen Druckluftsteuerungsvorrichtung;
- Fig. 2a,b: eine zeitliche Darstellung erfassten Drucks im Hohlkörper und des entsprechend modulierten Drucks in der Drückzuführungsleitung; und
- Fig. 3: eine schematische Darstellung der aus der WO 99/24101 bekannten Vorrichtung zur Erzeugung eines kontinuierlichen positiven Atemwegdrucks.

### BESCHREIBUNG DER AUSFÜHRUNGSBEISPIELE

In sämtlichen Figuren bezeichnen gleiche Bezugszeichen gleiche bzw. funktionsgleiche Komponenten.

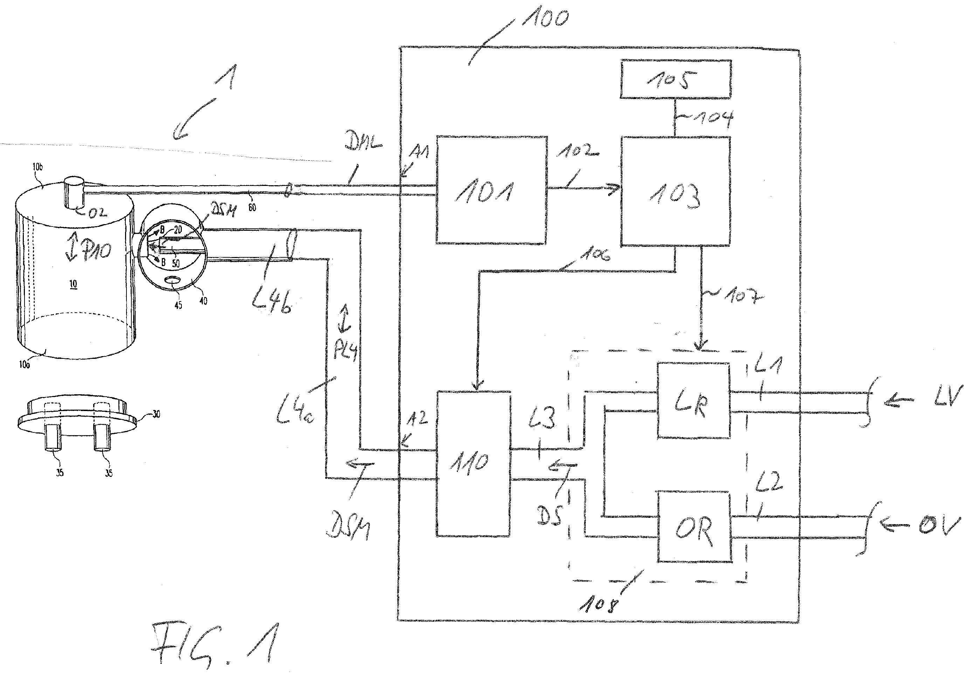

Fig. 1 ist eine schematische Darstellung einer Ausführungsform des erfindungsgemäßen CPAP-Systems mit einer entsprechenden Ausführungsform der erfindungsgemäßen Druckluftsteuerungsvorrichtung.

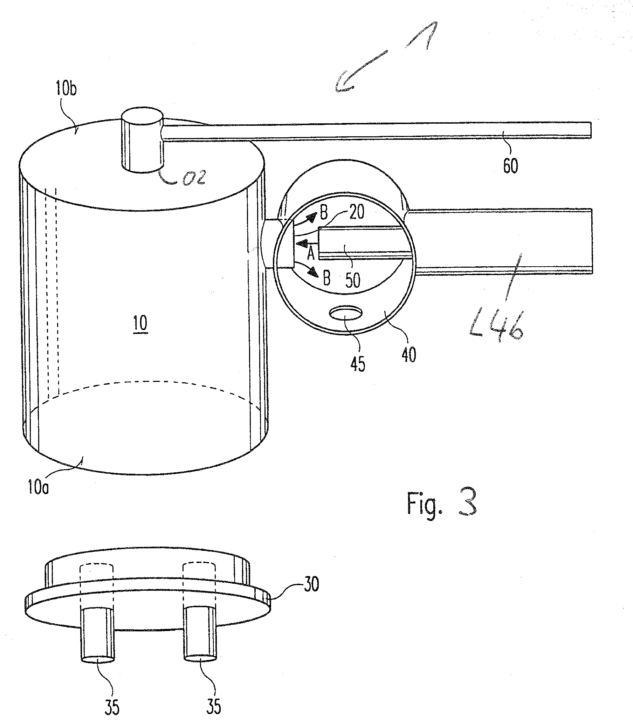

In Fig. 1 bezeichnet Bezugszeichen 1 eine CPAP-Vorrichtung, welche der bereits in Zusammenhang mit Fig. 3 beschriebenen, an sich bekannten, CPAP-Vorrichtung entspricht.

Zusätzlich wurde im Hohlkörper 10 das Bezugszeichen P10 eingeführt, welches einen zeitveränderlichen Druck im Hohlkörper bezeichnet, der sich über die zweite Öffnung 02 und die Röhre 60 erfassen lässt.

Weiterhin wird bei dieser erfindungsgemäßen Ausführungsform dem Hohlkörper 10 über die erste Öffnung 20 nicht mehr eine konstante Druckluftströmung A wie beim Stand der Technik von Fig. 3 zugeführt, sondern ein modulierter Druckluftstrom DSM, welcher von einer Druckluftsteuerungsvorrichtung 100 ansprechend auf den erfassten Druck P10 im Hohlkörper erzeugt wird.

Die Druckluftsteuerungsvorrichtung 100 weist einen ersten Anschluss A1 zum Anschließen einer Druckmessleitung DML auf, welche an die Röhre 60 der CPAP-Vorrichtung 1 angeschlossen ist.

Eine Druckerfassungseinrichtung 101, beispielsweise ein Drucksensor, empfängt über die Druckmessleitung DML den Druck P10 im Hohlkörper 10 der CPAP-Vorrichtung 1. Dabei ist zu bemerken, dass der erfasste Druck P10 unmittelbar die Atemaktivität des Patienten wiederspiegelt, da der Patient in den Hohlkörper 10 ein- bzw. ausatmet.

Der Drucksensor 101 erzeugt aus dem erfassten Druck P10 ein entsprechendes elektrisches Drucksignal 102, welches an eine Druckverlauf-Auswerteeinrichtung 103, 104, 105 ausgegeben wird, die eine Speichereinrichtung 105 umfasst, welche mit ihr über eine Leitung 104 verbunden ist.

Die Druckverlauf-Auswerteeinrichtung 103, 104, 105 wertet den vom Drucksensor 101 erfassten Druckverlauf des Drucks P10 gemäß mindestens einem vorbestimmten Kriterium aus und erzeugt ein Druckmodulations-Steuersignal 106 basierend auf dem Resultat des Auswertens.

Bei dieser Ausführungsform sind vorbestimmte Kriterien, beispielsweise Referenz-Druckverläufe, in der Speichereinrichtung 105 gespeichert, welche beim Auswerten zum Vergleichen herangezogen werden.

Der erfasste Druckverlauf und optional auch das Ergebnis der Auswertung können auf einer (nicht dargestellten) Anzeigeeinrichtung visuell dargestellt werden. Auch können optional akustische und/oder optische Warneinrichtungen für Gefahrensituationen, welche sich aus dem erfassten Druckverlauf ableiten lassen, vorgesehen werden.

Bezugszeichen 110 bezeichnet eine Druckmodulationsvorrichtung, welche einen im Wesentlichen konstanten Druckluftstrom DS empfängt und einen modulierten Druckluftstrom DSM an die CPAP-Vorrichtung 1 ausgibt, und zwar über den zweiten Ausgang A2, der über eine Druckversorgungsleitung L4a mit der Anschlussröhre L4b der CPAP-Vorrichtung verbunden ist.

Die Druckverlauf-Auswerteeinrichtung 103, 104, 105 erzeugt weiterhin ein Fluss-Steuersignal 107, welches an eine Fluss-Steuervorrichtung 108 geleitet wird, die der Druckmodulationsvorrichtung 110 vorgeschaltet ist.

Die Fluss-Steuervorrichtung 108 wird von einer (nicht dargestellten) Luft-Versorgungseinrichtung LV und von einer (nicht dargestellten) Sauerstoffversorgungseinrichtung (OV) gespeist, und ist derart gestaltet, dass durch sie neben dem Fluss des resultierenden konstanten Druckluftstrom DS, welcher der Druckmodulationsvorrichtung 110 zugeführt wird, auch ein Konzentrationsverhältnis von Luft und Sauerstoff einstellbar ist.

Die Fluss-Steuervorrichtung 108 weist intern eine Luftregelungseinrichtung LR und eine dazu parallel geschaltete Sauerstoffregelungseinrichtung OR auf, welche von den entsprechenden Versorgungseinrichtungen LV, OV durch Leitungen L1 bzw. L2 gespeist werden.

Die Ausgangsleitung der Fluss-Steuervorrichtung 108 zur Druckmodulationsvorrichtung 110 ist mit Bezugszeichen L3 bezeichnet.

Mit einer derart aufgebauten Druckluftsteuerungsvorrichtung für eine CPAP-Vorrichtung ist es möglich, der CPAP-Vorrichtung einen modulierten Druckluftstrom DSM zuzuführen, welcher beliebige Druckmuster aufweist, die sich über das erfasste Drucksignal im Hohlkörper 10 der CPAP-Vorrichtung 1 auslösen lassen.

Da die Aus- und Einatmung des Patienten in den Hohlkörper erfolgen, können aus dem erfassten Verlauf des Drucks P10 Informationen über die Atmung des Patienten extrahiert werden.

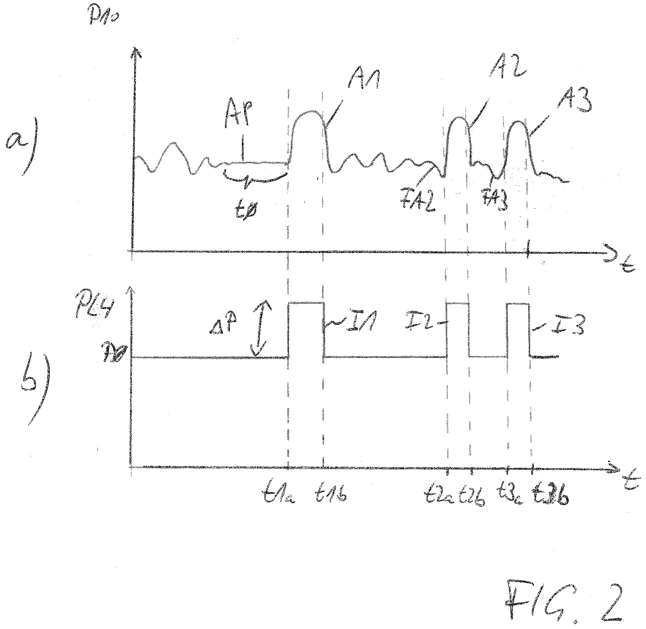

Durch die Analyse des dem Druckverlauf entsprechenden elektrischen Signals 102 in der Druckverlauf-Auswerteeinrichtung 103, 104, 105 kann auf spezielle Situationen in der Atmung durch bestimmte Druckmuster reagiert werden, was nachstehend mit Bezug auf Fig. 2 näher erläutert wird.

Fig. 2 ist eine zeitliche Darstellung erfassten Drucks im Hohlkörper und des entsprechend modulierten Drucks in der Druckzuführungsleitung.

In Fig. 2 bezeichnet t die Zeitachse, wobei in Fig. 2a) der zeitliche Verlauf des Drucks P10 im Hohlkörper 10 der CPAP-Vorrichtung 1 und in Fig. 2b) der Druck PL4 der Druckversorgungsleitung L4a der CPAP-Vorrichtung 1 dargestellt ist.

Mit Bezug auf Fig. 2a) wird zunächst von der Druckverlauf-Auswerteeinrichtung eine Apnoephase AP erfasst, welche sich dadurch äußert, dass der Druck P10 eine vorbestimmte Zeitdauer t0 von typischerweise 2 - 5s annähernd konstant ist. Nach Erfassen einer derartigen Apnoephase AP gibt die Druckverlauf-Auswerteeinrichtung 103, 104, 105 ein Druckmodulations-Steuersignal 106 an die Druckmodulationsvorrichtung 110 aus, welches ausgehend von einem Überdruck P0 einen Druckimpuls I1 initialisiert, der rechteckförmig ist und eine Amplitude ΔP aufweist. Die Zeitdauer des Druckimpulses I1 verläuft vom Zeitpunkt t1a zum Zeitpunkt t1b und bewirkt einen entsprechenden Druckimpuls A1 im Hohlkörper 10 der CPAP-Vorrichtung 1. Dieser Druckimpuls I1 bzw. der resultierende Druckimpuls A1 soll die spontane Atmung des Patienten stimulieren.

Im weiteren Zeitverlauf erfasst die Druckverlauf-Auswerteeinrichtung 103, 104, 105 eine abfallende Flanke FA2 des Drucks P10, welche dafür charakteristisch ist, dass an ihrem Ende ein spontaner Atemreflex des Patienten einsetzt.

Deshalb triggert die Druckverlauf-Auswerteeinrichtung 103, 104, 105 durch ein entsprechendes Druckmodulations-Steuersignal 106 die Druckmodulationsvorrichtung 110 zur Ausgabe eines Druckimpulses I2, welcher vom Zeitpunkt t2a bis zum Zeitpunkt t2b verläuft und ebenfalls eine Amplitude ΔP ausgehend vom Basisüberdruck P0 aufweist.

Wie in Fig. 2a) erkennbar, wird somit durch den Druckimpuls I2 das natürliche Atmen des Patienten, das sich im Druckimpuls A2 des Drucks P10 äußert, unterstützt.

Im Anschluss daran wird gemäß Fig. 2a) mit der abfallenden Flanke FA3 ein weiterer spontaner Atemreflex erfasst, der im Zeitfenster von t3a bis t3b durch einen entsprechenden Druckimpuls I3 der Druckmodulationsvorrichtung 110 unterstützt wird.

Obwohl die vorliegende Erfindung anhand eines bevorzugten Ausführungsbeispiels vorstehend beschrieben wurde, ist sie darauf nicht beschränkt, sondern auf vielfältige Weise modifizierbar.

Obwohl im obigen Ausführungsbeispiel eine Atemstimulation als Reaktion auf eine Apnoephase und eine Atemunterstützung korreliert zu natürlichen Atemreflexen dargestellt wurden, können selbstverständlich beliebige andere Druckmuster von der Druckmodulationsvorrichtung 110 an die CPAP-Vorrichtung ausgegeben werden. Dabei können selbstverständlich neben dem erfassten Druck P10 im Hohlkörper 10 auch andere Kriterien herangezogen werden, beispielsweise in die Drucksteuervorrichtung 100 eingegebene Patientenparameter.

Auch kann neben einer ereignisbezogenen Modulation auch eine Grundmodulation, beispielsweise ein periodisches Druckmuster, wie z. B. ein oszillierendes Sinusdruckmuster, in der Druckversorgungsleitung L4a vorgesehen werden.

## Patentansprüche

1. Druckluftsteuerungsvorrichtung für eine CPAP-Vorrichtung (1) mit:
einer Druckerfassungsvorrichtung (101) zum Erfassen eines Druckverlaufs des Drucks (P10) in einem Hohlkörper (10) der CPAP-Vorrichtung (1) und zum Erzeugen eines entsprechenden Drucksignals (102);
einer Druckverlauf-Auswerteeinrichtung (103, 104, 105) zum Auswerten des erfassten Druckverlaufs des Drucks (P10) gemäß mindestens einem vorbestimmten Kriterium und zum Erzeugen eines Druckmodulations-Steuersignals (106) basierend auf dem Resultat des Auswertens; und
einer Druckmodulationsvorrichtung (110) zum Empfangen eines im wesentlichen konstanten Druckluftstroms (DS) und zum Ausgeben eines modulierten Druckluftstroms (DSM) an die CPAP-Vorrichtung (1), welche derart gestaltet ist, dass sie ansprechend auf das Druckmodulations-Steuersignal (106) einen Druck (PL4) des an die CPAP-Vorrichtung (1) ausgegebenen Druckluftstroms (DSM) moduliert;
**dadurch gekennzeichnet, dass**
die Druckverlauf-Auswerteeinrichtung (103, 104, 105) derart gestaltet ist, dass sie beim Auswerten eine Apnoephase erfasst und ansprechend auf eine derartige Erfassung ein Druckmodulations-Steuersignal (106) zum Auslösen eines Druckimpulses (I1) im modulierten Druckluftstrom (DSM) an die Druckmodulationsvorrichtung (110) ausgibt;
die Druckverlauf-Auswerteeinrichtung (103, 104, 105) zum Erkennen einer Apnoephase erfasst, ob der Druck (P10) eine vorbestimmte Zeitdauer (t0) im wesentlichen konstant ist, wobei die vorbestimmte Zeitdauer (t0), vorzugsweise im Bereich von 2 bis 5s liegt; und
die Druckverlauf-Auswerteeinrichtung (103, 104, 105) derart gestaltet ist, dass sie beim Auswerten natürliche Atemreflexe erfasst und ansprechend auf eine derartige Erfassung ein Druckmodulations-Steuersignal (106) zum Auslösen eines Druckimpulses (I2, I3) im modulierten Druckluftstrom (DSM) an die Druckmodulationsvorrichtung (110) ausgibt, welcher mit dem natürlichen Atemreflex korreliert und synchronisiert ist.

2. Druckluftsteuerungsvorrichtung nach Anspruch 1, wobei die Druckverlauf-Auswerteeinrichtung (103, 104, 105) zum Erzeugen eines Fluß-Steuersignals (107) basierend auf dem Resultat des Auswertens gestaltet ist und eine Fluß-Steuervorrichtung (108) vorgesehen ist, welche derart gestaltet ist, dass sie ansprechend auf das Fluß-Steuersignal (107) einen Fluß des im wesentlichen konstanten Druckluftstroms (DS) steuert.

3. Druckluftsteuerungsvorrichtung nach Anspruch 1 oder 2, wobei die Druckverlauf-Auswerteeinrichtung (103, 104, 105) eine Speichereinrichtung (105) umfasst, in der Referenz-Druckverläufe gespeichert sind, und die Druckverlauf-Auswerteeinrichtung (103, 104, 105) derart gestaltet ist, dass sie beim Auswerten ein Vergleichen mit den Referenz-Druckverläufen durchführt.

4. Druckluftsteuerungsvorrichtung nach Anspruch 2, wobei die Fluß-Steuervorrichtung (108) von einer Luft-Versorgungseinrichtung (LV) und von einer Sauerstoffversorgungseinrichtung (OV) gespeist ist und derart gestaltet ist, dass durch sie ein Konzentrationsverhältnis von Luft und Sauerstoff einstellbar ist.

5. Druckluftsteuerungsvorrichtung nach Anspruch 1, wobei die Druckverlauf-Auswerteeinrichtung (103, 104, 105) zum Erkennen des Einsetzens natürlicher Atemreflexe erfasst, ob der Druck (P10) eine vorbestimmte abfallende Flanke (FA2, FA3) aufweist.

6. Druckluftsteuerungsvorrichtung nach Anspruch 1, wobei die Druckmodulationsvorrichtung (110) derart gestaltet ist, dass durch sie eine vorgebbare Grundmodulation im modulierten Druckluftstrom (DSM) zusätzlich vorsehbar ist, insbesondere eine periodische Modulation.

7. Druckluftsteuerungsvorrichtung nach Anspruch 1, wobei der jeweilige Druckimpuls (I1; I2, I3) im wesentlichen rechteckförmig ist.

8. CPAP-System mit einer Druckluftsteuerungsvorrichtung nach mindestens einem der vorhergehenden Ansprüche und einer CPAP-Vorrichtung (1), welche aufweist:
eine in einer Seitenwand des Hohlkörpers (10) vorgesehene erste Öffnung (20) zum Zuführen des modulierten Druckluftstroms (DSM) und zum Abführen eines ausgeatmeten Luftstroms (B) ;
ein am Hohlkörper (10) anbringbares Anschlußstück (30) zur Verbindung des Hohlkörpers (10) mit einem Nasenansatzstück (100);
eine Strömungsdüse (50) zum Richten des modulierten Druckluftstroms (DSM) auf die erste Öffnung (20); und
eine in einer Seitenwand des Hohlkörpers (10) vorgesehene zweite Öffnung (02) zum Anschließen der Druckerfassungsvorrichtung (101).

9. CPAP-System nach Anspruch 8, **dadurch gekennzeichnet, dass** der Hohlkörper (10) im wesentlichen die Gestalt eines Hohlzylinders aufweist, an dessen einer Stirnfläche (10a) das Anschlußstück (30) anbringbar ist und an dessen Mantelfläche die Öffnung (20) vorgesehen ist.

10. CPAP-System nach Anspruch 9, **dadurch gekennzeichnet, dass** die zweite Öffnung (02)in einer von der Seitenwand mit der ersten Öffnung (20) verschiedenen Seitenwand des Hoh1-körpers (10) vorgesehen ist.

11. CPAP-System nach Anspruch 10, **dadurch gekennzeichnet, dass** der Hohlkörper (10) im wesentlichen die Gestalt eines Hohlzylinders aufweist, an dessen einer Stirnfläche (10a) das Anschlußstück (30) anbringbar ist, an dessen anderer Stirnfläche (10b) die zweite Öffnung vorgesehen ist und an dessen Mantelfläche die erste Öffnung (20) vorgesehen ist.

12. CPAP-System nach Anspruch 11, **dadurch gekennzeichnet, dass** mit der zweiten Öffnung (02) unter einem im wesentlichen rechtem Winkel eine Röhre (60) zum Anschluß an eine Druckmeßleitung (DML) verbunden ist.

## Claims

1. Compressed air control device for a CPAP device (1) having:
a pressure ascertaining device (101) for ascertaining a pressure curve of the pressure (P10) in a hollow body (10) of the CPAP device (1) and for generating a corresponding pressure signal (102),
a pressure curve-evaluating means (103, 104, 105) for evaluating the ascertained pressure curve of the pressure (P10) in accordance with at least one predetermined criterion and for generating a pressure modulation control signal (106) based upon the result of the evaluation, and
a pressure modulation device (110) for receiving an essentially constant compressed air flow (DS) and for outputting a modulated compressed air flow (DSM) to the CPAP device (1), said pressure modulation device being embodied in such a manner that it modulates in response to the pressure modulation control signal (106) a pressure (PL4) of the compressed air flow (DSM) that is output to the CPAP device (1),
**characterized in that**
the pressure curve-evaluating means (103, 104, 105) is embodied in such a manner that during the evaluation procedure an apnoea phase is ascertained and in response to ascertaining said apnoea phase in this manner a pressure modulation control signal (106) is output so as to trigger a pressure pulse (I1) in the modulated compressed air flow (DSM) to the pressure modulation device (110),
the pressure curve-evaluating means (103, 104, 105) for identifying an apnoea phase ascertains whether the pressure (P10) is essentially constant over a predetermined time period (t0), wherein the predetermined time period (t0) is preferably in the region of 2 to 5 seconds, and
the pressure curve-evaluating means (103, 104, 105) is embodied in such a manner that during the evaluation procedure it ascertains natural breathing reflexes and in response to ascertaining said breathing reflexes in this manner outputs a pressure modulation control signal (106) so as to trigger a pressure pulse (12, 13) in the modulated compressed air flow (DSM) to the pressure modulation control device (110), said pulse being correlated and synchronised with the natural breathing reflex.

2. Compressed air control device according to Claim 1,
wherein the pressure curve-evaluating means (103, 104, 105) is embodied so as to generate a flow control signal (107) based upon the result of the evaluation procedure, and a flow control device (108) is provided that is embodied in such a manner that it controls a flow of the essentially constant compressed air flow (DS) in response to the flow control signal (107).

3. Compressed air control device according to Claim 1 or 2,
wherein the pressure curve-evaluating means (103, 104, 105) comprises a storage device (105) in which reference pressure curves are stored and the pressure curve-evaluating means (103, 104, 105) is embodied in such a manner that during the evaluation procedure it performs a comparison with the reference pressure curves.

4. Compressed air control device according to Claim 2,
wherein the flow control device (108) is supplied by an air supply unit (LV) and by an oxygen supply unit (OV) and is embodied in such a manner that it is possible to set a concentration ratio of air and oxygen.

5. Compressed air control device according to Claim 1,
wherein the pressure curve-evaluating means (103, 104, 105) for identifying the onset of natural breathing reflexes ascertains whether the pressure (P10) comprises a predetermined trailing edge (FA2, FA3).

6. Compressed air control device according to Claim 1,
wherein the pressure modulating device (110) is embodied in such a manner that it renders it possible to perform an additional predeterminable basic modulation procedure in the modulated compressed air flow (DSM), said additional predeterminable basic modulation procedure being in particular a periodic modulation procedure.

7. Compressed air control device according to Claim 1,
wherein the respective pressure pulse (I1, I2, I3) is essentially square.

8. CPAP system having a compressed air control device according to at least one of the preceding claims and a CPAP device (1) that comprises:
a first aperture (20) that is provided in a side wall of the hollow body (10) for supplying the modulated compressed air flow (DSM) and for dissipating an air flow (B) that is exhaled,
a connecting piece (30) that can be attached to the hollow body (10) so as to connect the hollow body (10) to a nose attachment piece (100),
a flow nozzle (50) for directing the modulated compressed air flow (DSM) to the first opening (20), and
a second aperture (02) that is provided in a side wall of the hollow body (10) so as to connect the pressure ascertaining device (101).

9. CPAP system according to Claim 8, **characterized in that** the hollow body (10) is essentially a hollow cylinder shape and it is possible to attach the connecting piece (30) to an end surface (10a) of said hollow body and the aperture (20) is provided on the peripheral surface of said hollow body.

10. CPAP system according to Claim 9, **characterized in that** the second aperture (02) is provided in a different side wall of the hollow body (10) to the sidewall having the first opening (20).

11. CPAP system according to Claim 10, **characterized in that** the hollow body (10) is essentially a hollow cylinder shape and it is possible to attach the connecting piece (30) to an end surface (10a) of said hollow body and the second aperture is provided on the other end surface (10b) of said hollow body and the first aperture (20) is provided on the peripheral surface of said hollow body.

12. CPAP system according to Claim 11, **characterized in that** a pipe (60) is connected at essentially a right angle to the second aperture (02) so as to connect to a pressure measuring line (DML).

## Revendications

1. Dispositif de commande à air comprimé pour un dispositif CPAP (1), comprenant :
un dispositif de detection de la pression (101) destiné à détecter une variation de la pression (P10) dans un corps creux (10) du dispositif CPAP (1) et destiné à générer un signal de pression (102) correspondant ;
un dispositif d'évaluation (103, 104, 105) de la variation de la pression destiné à évaluer la variation détectée de la pression (P10) selon au moins un critère prédéterminé et destiné à générer un signal de commande (106) de modulation de la pression sur la base du résultat de l'évaluation ; et
un dispositif de modulation de la pression (110) destiné à recevoir un courant d'air comprimé (DS) essentiellement constant et destiné à délivrer un courant d'air comprimé modulé (DSM) au dispositif CPAP (1), lequel est conçu de manière à ce qu'en réponse au signal de commande (106) de modulation de la pression, il module une pression (PL4) du courant d'air comprimé (DSM) délivré au dispositif CPAP ;
**caractérisé en ce que**
le dispositif d'évaluation (103, 104, 105) de la variation de la pression est conçu de manière à ce qu'il détecte une phase d'apnée lors de l'évaluation et qu'en réponse à une telle détection, il délivre au dispositif de modulation de la pression (110) un signal de commande (106) de modulation de la pression destiné à déclencher une impulsion de pression (11) dans le courant d'air comprimé modulé (DSM) ;
**en ce que** le dispositif d'évaluation (103, 104, 105) de la variation de la pression, dans le but d'identifier une phase d'apnée, détecte si la pression (P10) est essentiellement constante pendant une durée prédéterminée (t0), la durée prédéterminée (t0) étant située de préférence dans la plage de 2 à 5 s ; et
**en ce que** le dispositif d'évaluation (103, 104, 105) de la variation de la pression est conçu de manière à ce qu'il détecte des réflexes respiratoires naturels lors de l'évaluation et qu'en réponse à une telle détection, il délivre au dispositif de modulation de la pression (110) un signal de commande (106) de modulation de la pression destiné à déclencher une impulsion de pression (I2, I3) dans le courant d'air comprimé modulé (DSM), laquelle est corrélée avec le réflexe respiratoire naturel et y est synchronisée.

2. Dispositif de commande à air comprimé selon la revendication 1, dans lequel le dispositif d'évaluation (103, 104, 105) de la variation de la pression est conçu pour générer un signal de commande de flux (107) sur la base du résultat de l'évaluation et en ce qu'un dispositif de commande de flux (108) est ménagé, lequel est conçu de manière à ce qu'en réponse au signal de commande de flux (107), il commande un flux du courant d'air comprimé (DS) essentiellement constant.

3. Dispositif de commande à air comprimé selon la revendication 1 ou 2, dans lequel le dispositif d'évaluation (103, 104, 105) de la variation de la pression comprend un moyen de mémorisation (105) dans lequel des variations référentielles de la pression sont mémorisés, et en ce que le moyen d'évaluation (103, 104, 105) de la variation de la pression est conçu de manière à ce que, lors de l'évaluation, il réalise une comparaison avec les variations référentielles de la pression.

4. Dispositif de commande à air comprimé selon la revendication 2, dans lequel le dispositif de commande du flux (108) est alimenté par un moyen d'alimentation en air (LV) et par un moyen d'alimentation en oxygène (OV) et est conçu de manière à ce qu'il est susceptible de régler un rapport de concentration de l'air et de l'oxygène.

5. Dispositif de commande à air comprimé selon la revendication 1, dans lequel le moyen d'évaluation (103, 104, 105) de la variation de la pression, dans le but d'identifier le commencement des réflexes respiratoires naturels, détecte si la pression (P10) présente un flanc descendant prédéterminé (FA2, FA3).

6. Dispositif de commande à air comprimé selon la revendication 1, dans lequel le dispositif de modulation (110) de la pression est conçu de manière à ce qu'il est susceptible de prévoir une modulation de base prédéfinissable en plus dans le courant d'air comprimé modulé (DSM), notamment une modulation périodique.

7. Dispositif de commande à air comprimé selon la revendication 1, dans lequel l'impulsion de pression (I1 ; I2, I3) respective est essentiellement rectangulaire.

8. Système CPAP comportant un dispositif de commande à air comprimé selon au moins l'une des revendications précédentes et un dispositif CPAP (1), lequel présente :
une première ouverture (20) ménagée dans une paroi latérale du corps creux (10), destinée à amener le courant d'air comprimé modulé (DSM) et à évacuer un courant d'air respiré (B) ;
une pièce de raccordement (30) pouvant être installée sur le corps creux (10), destinée au raccordement du corps creux (10) à un embout nasal (100) ;
une buse d'écoulement (50) destinée à orienter le courant d'air comprimé modulé (DSM) vers la première ouverture (20) ; et
une deuxième ouverture (02) ménagée dans une paroi latérale du corps creux (10), destinée au raccordement du dispositif de détection de la pression (101).

9. Système CPAP selon la revendication 8, **caractérisé en ce que** le corps creux (10) présente essentiellement la configuration d'un cylindre creux sur une surface frontale (10a) duquel la pièce de raccordement (30) peut être installée et sur la surface d'enveloppe duquel est ménagée l'ouverture (20).

10. Système CPAP selon la revendication 9, **caractérisé en ce que** la deuxième ouverture (02) est ménagée dans une paroi latérale du corps creux (10), différente de la paroi latérale comportant la première ouverture (20).

11. Système CPAP selon la revendication 10, **caractérisé en ce que** le corps creux (10) présente essentiellement la configuration d'un cylindre creux sur une surface frontale (10a) duquel la pièce de raccordement (30) peut être installée, sur l'autre surface frontale (10b) duquel est ménagée la deuxième ouverture (02) et sur la surface d'enveloppe duquel est ménagée la première ouverture (20)

12. Système CPAP selon la revendication 11, **caractérisé en ce qu'**un tuyau (60) destiné au raccordement à une ligne de mesure de pression (DML) est raccordé à la deuxième ouverture (02) dans un angle essentiellement droit.
